## Europäisches Patentamt
## European Patent Office
## Office européen des brevets

(19)

(11) Veröffentlichungsnummer: **0 242 719**
**B1**

(12) **EUROPÄISCHE PATENTSCHRIFT**

(45) Veröffentlichungstag der Patentschrift:
**29.08.90**

(21) Anmeldenummer: **87105262.7**

(22) Anmeldetag: **09.04.87**

(51) Int. Cl.⁵: **A61F 2/34**

(54) **Künstliche Hüftgelenkspfanne.**

(30) Priorität: **17.04.86 CH 1553/86**

(43) Veröffentlichungstag der Anmeldung:
**28.10.87 Patentblatt 87/44**

(45) Bekanntmachung des Hinweises auf die Patenterteilung:
**29.08.90 Patentblatt 90/35**

(84) Benannte Vertragsstaaten:
**AT DE FR GB IT**

(56) Entgegenhaltungen:
**EP-A- 0 190 422**
**DE-A- 3 027 063**
**DE-A- 3 228 113**
**GB-A- 2 059 267**
**US-A- 3 938 198**

(73) Patentinhaber: **GEBRÜDER SULZER AKTIENGESELLSCHAFT, Zürcherstrasse 9, CH-8401 Winterthur(CH)**

(72) Erfinder: **Griss, Peter, Prof. Dr.-med., Orthopädische Klinik am Klinkum Philipps-Univ., Baldingerstrasse D-3550 Marburg(DE)**

(74) Vertreter: **Sparing Röhl Henseler Patentanwälte European Patent Attorneys, Rethelstrasse 123, D-4000 Düsseldorf 1(DE)**

ACTORUM AG

## Beschreibung

Die Erfindung betrifft eine künstliche Hüftgelenkspfanne, bestehend aus einem aus Kunststoff gefertigten rotationssymmetrischen Pfannenkörper, der die eigentliche Pfannenschale enthält, und aus einem Metallgitter, das in der äusseren Oberfläche des Pfannenkörpers verankert ist.

Eine Hüftgelenkspfanne der vorstehend genannten Art ist bereits vorgeschlagen worden (CH-A 655 770 und daraus resultierende EP-Anmeldung 85 115 115.9, veröffentlicht als EP-A 0 190 422 und Stand der Technik nach Artikel 54 (3) EPÜ). Bei dieser Pfanne ist in die Aussenfläche eines Kunststoffpfannenkörpers ein mehrlagiges Drahtnetz eingepresst, wobei eine Lage des Netzes zur innigen Verbindung beider in den Kunststoff eingebettet ist. In die restlichen Lagen des Netzes, die eine poröse "Aussenhaut" der Pfanne bilden, soll zur dauerhaften Verankerung im Knochen Gewebe einwachsen. Bevor die dauerhafte Verankerung durch einwachsendes Gewebe erfolgt, wird bei dieser Pfanne die Primärfixierung dadurch erzeugt, dass die Pfanne im Beckenknochen unter einer Vorspannung steht (CH-A 666 609).

Aufgabe der Erfindung ist es, diese Primärfixierung der vorstehend beschriebenen Pfanne zu verbessern und eine elastische Verbindung der netzverstärkten Pfanne mit dem Knochen zu schaffen. Diese Aufgabe wird nach der Erfindung dadurch gelöst, dass an dem Metallgitter symmetrisch zu einer Meridianebene von Pfannenschale und Pfannenkörper eine Vielzahl von aus dem äquatorialen Rand hervorstehenden Laschen vorhanden ist, die intraoperativ bleibend verformbar sind und mindestens zum Teil Durchtrittsbohrungen für Knochenschrauben oder -nägel aufweisen.

Mit Hilfe der Laschen, die intraoperativ verformt oder auch teilweise abgetrennt werden können, ist es möglich, die Pfanne direkt bei der Implantation im Beckenknochen mit Nägeln und/oder Schrauben zu fixieren, wobei dadurch, dass die Laschen ein Teil des Netzes sind, die elastische Verformbarkeit der Pfanne nicht unzulässig beeinträchtigt wird.

Infolge des symmetrischen Aufbaues und der intraoperativen "Abtrennbarkeit" einzelner Laschen ist es möglich, den gleichen Pfannentyp für rechte und für linke Gelenke zu verwenden, und auch Anforderungen hinsichtlich unterschiedlicher örtlicher "Einsatz"-Punkte und -richtungen für die Schrauben oder Nägel zu entsprechen. Daher resultiert aus diesen beiden Eigenschaften eine erhebliche Vereinfachung für die Fabrikation und die Lagerhaltung. Weiterhin können dadurch die örtlichen Fixierungspunkte im Beckenknochen relativ frei gewählt werden.

Dies ist besonders wichtig für sogenannte "Reoperations"-Pfannen, die bei Reoperationen dazu dienen, eine andere, früher eingesetzte künstliche Hüftgelenkspfanne zu ersetzen. Besonders im Hinblick auf den Reoperationsfall können die Laschen unterschiedliche radiale Längen und/oder mehrere, in radialer Richtung hintereinander liegende Durchtrittsbohrungen haben.

Im folgenden wird die Erfindung anhand eines Ausführungsbeispiels im Zusammenhang mit der Zeichnung näher beschrieben.

Fig. I zeigt schematisch eine Aufsicht auf die neue Pfanne in Richtung der Pfannenachse, während

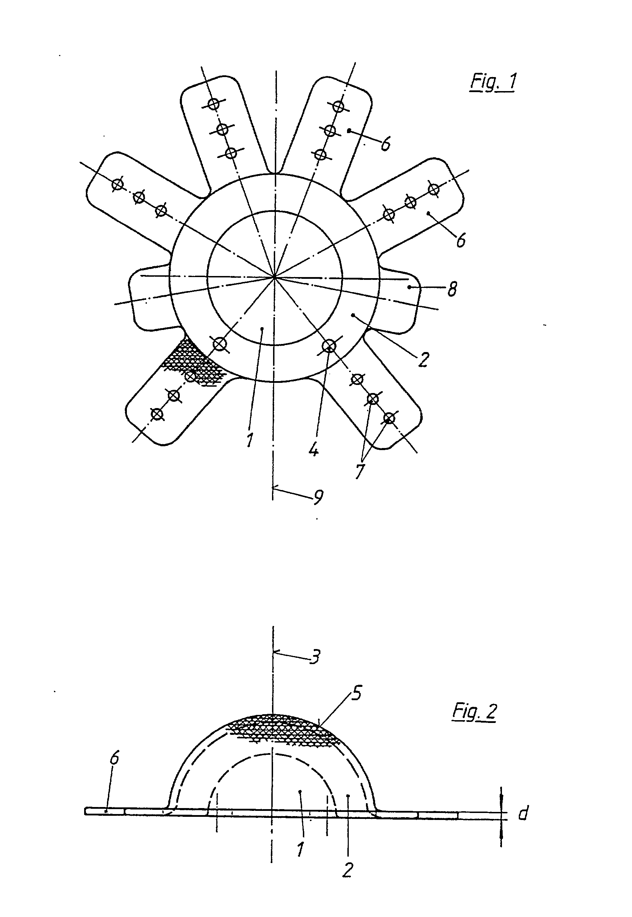

Fig. 2 eine Seitenansicht widergibt.

Die den nicht gezeigten Gelenkkopf einer Femurkopfprothese aufnehmende halbkugelförmige Pfannenschale I befindet sich in einem Pfannenkörper 2 aus Kunststoff, beispielsweise aus Polyäthylen. Pfannenschale I und Pfannenkörper 2 sind rotationssymmetrisch zur Pfannenachse 3(Fig. 2) ausgebildet. Durch den Pfannenkörper 2 verlaufen Bohrungen 4, durch die Knochenschrauben oder Nägel in den Beckenknochen eingetrieben werden können.

Auf die Aussenfläche des Pfannenkörpers 2 ist als Verstärkungselement, das zugleich zur Verankerung der Pfanne im Beckenknochen dient, ein Metallgitter in Form eines mehrlagigen Drahtnetzes 5 aufgebracht. Pfannenkörper 2 und Drahtnetz 5 sind dadurch fest miteinander verbunden, dass die innerste Lage des Netzes 5 in den Kunststoff des Pfannenkörpers 2 eingepresst ist. Erfindungsgemäss weist dieses Netz 5 an seinem äquatorialen Rand eine Vielzahl Laschen 6 und 8 auf, die teilweise ebenfalls mit Bohrungen 7 versehen sind. Die Dicke d (Fig. 2) des Netzes 5 bzw. der Laschen 6 und 8 ist so gewählt, dass sie intraoperativ bleibend verformt und an die Auflagefläche auf dem Beckenknochen angepasst sowie ein Teil von ihnen ohne Mühe abgetrennt werden können.

Um eine Verwendung der gleichen Pfanne sowohl in der linken als auch in der rechten Hüfte zu ermöglichen, sind die Laschen 6 und 8 - sowie die Bohrungen 4 im Pfannenkörper 2 - symmetrisch zu einer Ebene 9 durch die Pfannenachse 3 auf dem Rand der Pfanne bzw. des Netzes 5 verteilt.

Selbstverständlich kann die neue Pfanne nicht nur für zementfreie Implantationen dienen, sondern auch in Verbindung mit Knochenzement verwendet werden, wobei eine mechanische "Verzahnung" zwischen dem Metallgitter und dem Knochenzement entsteht.

## Patentansprüche

I. Künstliche Hüftgelenkspfanne, bestehend aus einem aus Kunststoff gefertigten rotationssymmetrischen Pfannenkörper (2), der die eigentliche Pfannenschale (1) enthält, und aus einem Metallgitter (5), das in der äusseren Oberfläche des Pfannenkörpers (2) verankert ist, wobei an dem Metallgitter (5) symmetrisch zu einer Meridianebene (9) von Pfannenschale (I) und Pfannenkörper (2) eine Vielzahl von aus dem äquatorialen Rand hervorstehenden Laschen (6, 8) vorhanden ist, die intraoperativ bleibend verformbar sind und mindestens zum Teil Durchtrittsbohrungen (7) für Knochenschrauben oder -nägel aufweisen.

2. Hüftgelenkspfanne nach Anspruch I, dadurch gekennzeichnet, dass die Laschen (6, 8) unterschiedliche radiale Längen haben.

3. Hüftgelenkspfanne nach Anspruch I oder 2, dadurch gekennzeichnet, dass einzelne Laschen (6) mehrere, in Radialrichtung hintereinander liegende Durchtrittsbohrungen (7) haben.

## Claims

1. A replacement acetabulum comprising: a plastics rotationally symmetrical socket receiving the actual acetabulum shell; and a metal mesh anchored in the outside surface of the socket, characterised in that a plurality of tabs (6, 8), projecting from the equatorial edge are present on the metal mesh (5) symmetrically of a meridian plane (9) of the shell (1) and socket (2), are permanently deformable intraoperatively and are formed at least to some extent with through bores (7) for bone screws or bone nails.

2. A replacement acetabulum according to claim 1, characterised in that the tabs (6, 8) are of different radial lengths.

3. A replacement acetabulum according to claim 1 or 2, characterised in that discrete tabs (6) are formed with number of radially consecutive through bores (7).

## Revendications

1. Cuvette artificielle pour articulation coxofémorale, comprenant un corps de cuvette à symétrie de rotation, fabriqué en matière plastique et renfermant la calotte de cuvette proprement dite, ainsi qu'un tressage métallique qui est ancré dans la surface extérieure du corps de cuvette, caractérisée par la présence sur le tressage métallique (5), symétriquement par rapport à un plan méridien (9) de la calotte de cuvette (1) et du corps de cuvette (2), d'un grand nombre de pattes (6, 8) qui font saillie au-delà du bord équatorial, sont déformables durablement par voie intraopératoire et présentent, au moins en partie, des orifices de passage (7) pour des vis ou des broches implantées dans l'os.

2. Cuvette pour articulation coxofémorale selon la revendication 1, caractérisée par le fait que les pattes (6, 8) présentent des longueurs radiales différentes.

3. Cuvette pour articulation coxofémorale selon la revendication 1 ou 2, caractérisée par le fait que des pattes individuelles (6) comportent plusieurs orifices de passage (7) agencés les uns derrière les autres dans le sens radial.

EP 0 242 719 B1

_Fig. 1_

_Fig. 2_